# NEW EUROPEAN PATENT SPECIFICATION

(11) **EP 2 641 628 B2**
(45) Date of publication and mention of the opposition decision: **01.05.2019**
(45) Mention of the grant of the patent: 29.07.2015
(21) Application number: 11841088.5
(22) Date of filing: 16.11.2011
(51) Int. Cl.: A61M 5/315

(54) **TWO-CHAMBERED CONTAINER-CUM-SYRINGE AND SYRINGE-FILLED ARIPIPRAZOLE**
ZWEIKAMMERBEHÄLTER MIT SPRITZE UND IN EINE SPRITZE GEFÜLLTES ARIPIPRAZOL
CONTENANT AVEC SERINGUE À DEUX CHAMBRES ET SERINGUE REMPLIE D'ARIPIPRAZOLE

(30) Priority: 16.11.2010 JP 2010256188
(43) Date of publication of application: 25.09.2013
(73) Proprietor: ARTE CORPORATION, Shinagawa-ku, Tokyo 140-0011 (JP); Otsuka Pharmaceutical Co., Ltd., Tokyo 101-8535 (JP)
(72) Inventor: KAKIUCHI Makoto, Takahagi-shi Ibaraki 318-0004 (JP); SHIMAZAKI Seiji, Takahagi-shi Ibaraki 318-0004 (JP); TAKESHIMA Yasuhiko, Takahagi-shi Ibaraki 318-0004 (JP); HIRAOKA Shogo, Osaka-shi Osaka 541-0045 (JP); MAESAKA Tadayoshi, Osaka-shi Osaka 541-0045 (JP)
(74) Representative: Hoffmann Eitle
(86) International application number: PCT/JP2011/076385
(87) International publication number: WO 2012/067141

(56) References cited:
- EP-A2- 0 568 321
- EP-A2- 2 444 110
- WO-A1-95/17916
- WO-A1-2011/059042
- WO-A2-2005/041937
- AU-B2- 560 426
- JP-A- H08 503 160
- JP-A- 2010 256 188
- JP-A- 2012 085 813
- JP-B1- 4 757 951
- JP-B2- 2 514 472
- US-A- 4 874 381

## Description

The present invention relates to a dual chamber prefillable syringe.

Prefillable syringes are filled with a liquid preparation in advance, and thus these can be used promptly with no troublesome operation after being taken out from the packaging in medical institutions. As described above, prefillable syringes are excellent in terms of convenience and significantly contribute to the reduction in workload of people related to medical service such as doctors and nurses, and thus these are employed in many hospitals.

Hitherto, as a type of the prefillable syringe, dual chamber prefillable syringes have been known in which a preparation and a solvent or solution (dispersion medium) are separately filled.

The dual chamber prefillable syringe has a front stopper inserted into the front end side of a cylinder and an end stopper inserted into the rear end side of the cylinder, and a middle stopper inserted into the central part of the inside of the cylinder, the cylinder is divided into two front and rear chambers. In addition, a bypass portion which is formed so that the inner circumferential surface of the cylinder partially bulges to the outside is formed at a part positioned closer to the front end than the middle stopper of the cylinder. Furthermore, a powder is sealed in the front chamber on the front end side of the middle stopper, and the front end is sealed by the front stopper. A solvent is sealed in the rear chamber on the rear end side of the middle stopper, and the rear end is sealed by an end stopper. A plunger rod is connected to the rear end of the end stopper.

When the dual chamber prefillable syringe having such a configuration is used, the end stopper is advanced in the cylinder by pressing the plunger rod into the cylinder. Therefore, the pressing force by the advance of the end stopper is transmitted to the middle stopper via the solvent, and thus the middle stopper also advances with the advance of the end stopper. When the middle stopper reaches the bypass portion, the front chamber and the rear chamber communicate with each other via the bulging part of the bypass portion. Accordingly, the solvent in the rear chamber flows into the front chamber and is mixed with the preparation in the front chamber, and thus the injection is reconstituted.

Aripiprazole which is used as an active component of a pharmaceutical composition is known as an atypical antipsychotic drug which is useful for treatment for a schizophrenic and is expressed by the following structural formula (for example, refer to U.S. Patent No. 5006528):

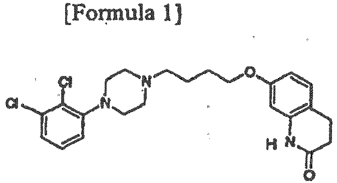

When a prefillable syringe containing aripiprazole, that is, aripiprazole filled in a syringe is used, for example, a cake composition is reconstituted which is obtained by suspending a pharmaceutical composition by a dispersion medium and then freeze-drying the suspension. When the cake composition is used, it is mixed with a desired dispersion medium (injectable liquid) to be resuspended, and the resuspension is injected to a patient intramuscularly or hypodermically (for example, refer to Japanese Patent Application, Publication No. 2007-509148).

In addition, Japanese Patent Application, Publication No. 8-112333 discloses a dual chamber prefillable syringe called a "two-chamber syringe" in which a freeze-dried material is reconstituted by freeze-drying a solution in the syringe and is then sealed by a rubber closure, and an injectable liquid is sealed in another partition chamber in the syringe.

In the above-described dual chamber prefillable syringe, in order to appropriately mix the solvent and the preparation, the middle stopper is required to be positioned in the bypass portion until the entire solvent in the rear chamber flows to the front chamber. In addition, the flow of the solvent is required to be controlled in the case of a preparation which is not easily dissolved.

However, when the plunger rod is pressed too early, or pressed excessively strongly without confirmation of the end of the flow of the solvent to the front chamber, the middle stopper moves to be closer to the front end than the bypass portion in a state in which the entire solvent does not completely flow to the front chamber. Therefore, the solvent in the rear chamber remains without being mixed with the preparation.

In this manner, due to the incorrect operation of the plunger rod, a useless solvent, which will not be mixed with the preparation, is generated, and as a result, problem occurs in that an injection having a predetermined concentration cannot be reconstituted and the preparation is not sufficiently dissolved by the solvent.

In the case of the above-described aripiprazole filled in a syringe, the residue caused by incomplete suspension in resuspending has become a problem.

It is desirable to overcome the above problems, for example by providing a dual chamber prefillable syringe in which a solvent and a preparation can be securely and appropriately mixed with each other in accordance with properties thereof.

It is desirable to overcome the above problems, for example by providing aripiprazole filled in a syringe which is adapted to reduce the residue caused by incomplete suspension in resuspending.

Japanese patent publication no. JP 2514472 B2 describes a two-compartment syringe and application process, but does not disclose the feature combination set forth in the characterizing portion of claim 1 below.

The invention provides a dual chamber prefillable syringe as recited in Claim 1.

In order that the invention will be more readily understood, embodiments thereof will now be described, by way of example only, in relation to the drawings, and in which:-
FIG. 1 is a longitudinal sectional view of a dual chamber prefillable syringe (aripiprazole filled in a syringe) according to an embodiment;
FIG 2A is a perspective view illustrating a finger grip;
FIG. 2B is a longitudinal sectional view illustrating a finger grip;
FIG. 3 is a side view of a plunger rod;
FIG. 4 is a side view of the plunger rod;
FIG. 5 is a perspective view of the plunger rod;
FIG 6A is a view illustrating a method of using the dual chamber prefillable syringe according to the embodiment;
FIG. 6B is a view illustrating a method of using the dual chamber prefillable syringe according to the embodiment;
FIG. 6C is a view illustrating a method of using the dual chamber prefillable syringe according to the embodiment;
FIG. 7A is a view illustrating a method of using the dual chamber prefillable syringe according to the embodiment;
FIG. 7B is a view illustrating a method of using the dual chamber prefillable syringe according to the embodiment;
FIG 8 is a perspective view of the finger grip and the plunger rod which is inserted into the inside of the finger grip;
FIG. 9A is a view of the finger grip of a modified embodiment; and
FIG. 9B is a view of the finger grip of a modified embodiment.

When a dual chamber prefillable syringe having the characteristics of an embodiment of the invention is used, the plunger rod can be rotated in accordance with the screwing of the male screw portion to the female screw portion. Accordingly, the plunger rod gradually advances in accordance with the pitches of the male screw portion and the female screw portion. Therefore, it is possible to avoid the excessive advancing speed of the plunger rod, and thus it is possible to allow the middle stopper to keep in the bypass portion. Accordingly, the flowing speed of the solvent to the front chamber can be easily adjusted and made constant, and it is possible to efficiently mix the preparation in the solvent.

In addition, in the dual chamber prefillable syringe according to an embodiment of the invention, when the middle stopper which is advanced by pressing the plunger rod enters into the bypass portion, the front end of the male screw portion preferably reaches the rear end of the female screw portion and can be screwed thereto.

When the middle stopper is advanced via the end stopper and the solvent by pressing the plunger rod, the front end of the male screw portion is brought into contact with the rear end of the female screw portion when the middle stopper enters into the bypass portion. Accordingly, even when the plunger rod is further pressed, the plunger rod does not advance anymore, and the middle stopper also does not advance. Therefore, it is possible to avoid the middle stopper from being excessively advanced up to the front end side of the bypass portion due to the excessive force applied to the plunge rod.

Moreover, after the front end of the male screw portion is brought into contact with the rear end of the female screw portion as described above, the plunger rod is gradually advanced in accordance with the screwing between the male screw portion and the female screw portion by rotating the plunger rod. When the middle stopper advances with the advance of the plunger rod and completely enters into the bypass portion, the solvent is introduced to the preparation via the bypass portion, and both of them are mixed. The flowing of the solvent to the preparation is adjusted by the pitches of the male screw portion and the female screw portion, and thus the speed can be adjusted to a speed at which the preparation is easily dissolved.

Accordingly, since the plunger rod can be rotated in accordance with the screwing between the male screw portion and the female screw portion only when the middle stopper reaches the bypass portion and the solvent is introduced to the preparation, only an operation of pressing the plunger rod may be performed until a part of the middle stopper reaches the bypass portion. Therefore, it is possible to avoid the handling of the dual chamber prefillable syringe from becoming troublesome.

The meaning of "when the middle stopper enters into the bypass portion" includes when the front end of the middle stopper reaches the rear end of the bypass portion, when a part of the middle stopper enters into the bypass portion, and immediately before the front end of the middle stopper reaches the rear end of the bypass portion.
That is, a configuration may be provided in which the front end of the male screw portion reaches the rear end of the female screw portion immediately before the front end of the middle stopper reaches the bypass portion.

Furthermore, the dual chamber prefillable syringe further includes: a guide groove which is formed in the female screw portion and extends parallel to the axis line; and a guide plate which is formed on the rear end side of the male screw portion on the outer circumferential surface of the plunger rod and is guided in accordance with the guide groove.

When the plunger rod is rotated and advanced in accordance with the screwing between the male screw portion and the female screw portion, the male screw portion of the plunger rod passes through the female screw portion of the finger grip, and thus the screwing between the male screw portion and the female screw portion is released. Accordingly, the plunger rod can be pressed. At this time, for example, the plunger rod is stopped at a position in which the guide plate of the plunger rod and the guide groove formed in the female screw portion can be fitted to each other. Therefore, by pressing the plunger rod, the plunger rod can be guided in the direction in which the guide groove extends. Accordingly, the plunger rod can be securely switched to going straight from the rotation, and by allowing the plunger rod to go straight, the discharge of bubbles in the cylinder and the injection of a liquid preparation to a patient can be securely performed.

Furthermore, the dual chamber prefillable syringe further includes: a first protrusion which is formed at the rear end of the finger grip and with which the guide plate is brought into contact so as to be able to surmount thereover when at least a part of the middle stopper which is advanced by rotating the plunger rod in accordance with the female screw portion and the male screw portion moves across the bypass portion.

When a part of the middle stopper advancing in the cylinder moves across the bypass portion by rotating and advancing the plunger rod in accordance with the screwing between the male screw portion and the female screw portion, the guide plate of the plunger rod is brought into contact with the first protrusion. Accordingly, a healthcare worker can easily recognize that since a part of the middle stopper moves across the bypass portion, the space between the middle stopper and the front stopper, that is, the space in which the solvent and the preparation are mixed is sealed again. At this time, the healthcare worker shakes the dual chamber prefillable syringe, and thus an injection can be reconstituted in which the preparation is completely mixed, dissolved or suspended in the solvent.

Furthermore, in the dual chamber prefillable syringe, after the guide plate surmounts the first protrusion by rotating the plunger rod in accordance with the female screw portion and the male screw portion, the screwing between the male screw portion and the female screw portion is preferably released.

Accordingly, after a part of the middle stopper moves across the bypass portion due to the rotation and advance of the plunger rod in accordance with the screwing between the male screw portion and the female screw portion, the plunger rod can be pressed. That is, since the plunger rod is rotated only when the solvent is introduced to the preparation, and then the operation is switched to an operation of pressing the plunger rod, it is possible to simplify the handling of the dual chamber prefillable syringe. In addition, since the guide plate is brought into contact with the first protrusion before the screwing between the male screw portion and the female screw portion is released, a healthcare worker can detect the end of the rotation of the plunger rod in advance.

In addition, the dual chamber prefillable syringe further includes: a second protrusion which is formed at the rear end of the finger grip and is brought into contact at a position in which the guide plate surmounting the first protrusion can be fitted to the guide groove with the guide plate.

When the guide plate surmounts the first protrusion as described above, the screwing between the male screw portion of the plunge rod and the female screw portion of the finger grip is released, and thus the advance due to the rotation of the plunger rod is switched to the advance due to the going straight. In addition, at this time, the guide plate is brought into contact with the second protrusion, and thus the healthcare worker recognizes that the plunger rod can be pressed. That is, the operation of the plunger rod can be securely switched by the contact of the guide plate with the second protrusion. Moreover, in this state, the guide plate and the guide groove can be fitted to each other. Accordingly, thereafter, by pressing the plunger rod, the plunger rod can be advanced along the guide plate.

Furthermore, the preparation preferably includes aripiprazole.

That is, although aripiprazole is difficult to dissolve in the solvent, the preparation can be appropriately dissolved in the solvent in the dual chamber prefillable syringe having the above-described configuration. Therefore, even when aripiprazole is employed as a preparation, the aripiprazole can be easily suspended in the solvent.

In addition, aripiprazole filled in a syringe according to an embodiment of the invention includes: aripiprazole; a cylinder which has a cylindrical shape with an axis line as a center and has a bypass portion formed due to bulging a part of an inner circumferential surface to the outside; a hub luer-lock which is provided at the front end of the cylinder; a finger grip which is provided at the rear end of the cylinder; a front stopper which is fitted on the front end side of the bypass portion in the cylinder; a middle stopper which is fitted on the rear end side of the bypass portion in the cylinder to seal the aripiprazole together with the front stopper; an end stopper which is fitted on the rear end side of the middle stopper in the cylinder to seal a solvent together with the middle stopper; a plunger rod which is connected to the end stopper from the rear end by inserting the finger grip; a female screw portion which is formed to be screwed around the axis line on an inner circumferential surface of the finger grip; and a male screw portion which is formed to be able to be screwed to the female screw portion on an outer circumferential surface of the plunger rod.

When the aripiprazole filled in a syringe according to an embodiment of the invention is used, the plunger rod is rotated in accordance with the screwing between the male screw portion and the female screw portion. Accordingly, the plunger rod gradually advances in accordance with the pitches of the male screw portion and the female screw portion. Therefore, it is possible to avoid the excessive advancing speed of the plunger rod, and thus it is possible to allow the middle stopper to stay in the bypass portion. Accordingly, the flowing speed of the solvent to the front chamber can be easily adjusted and made constant, and it is possible to reduce the residue in resuspending.

According to the dual chamber prefillable syringe of an embodiment of the invention, it is possible to avoid the excessive advancing speed of the plunger rod, and thus it is possible to prevent the middle stopper from being excessively advanced and moving out of the bypass portion. Accordingly, it is possible to allow the middle stopper to easily stay in the bypass portion, and the flowing speed of the solvent is adjusted by pitches of the mail screw portion and the female screw portion, whereby it is possible to appropriately mix the solvent and the preparation.

In addition, according to the aripiprazole filled in a syringe, it is possible to reduce the residue and incomplete dissolution in resuspending.

Hereinafter, a dual chamber prefillable syringe and aripiprazole filled in a syringe according to embodiments of the invention will be described in detail with reference to the drawings.

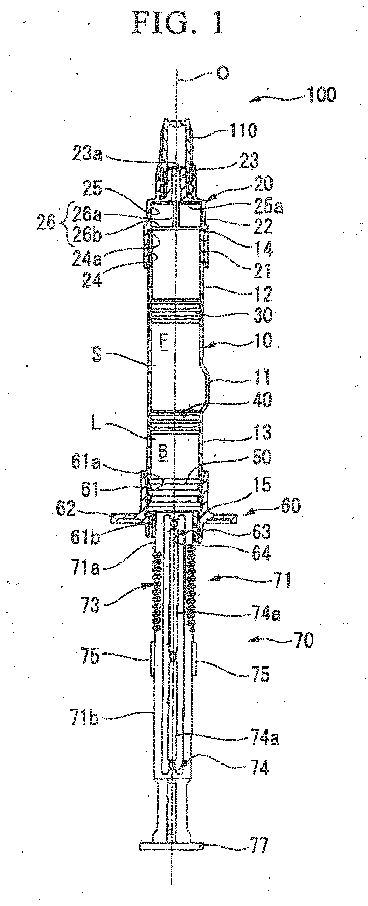

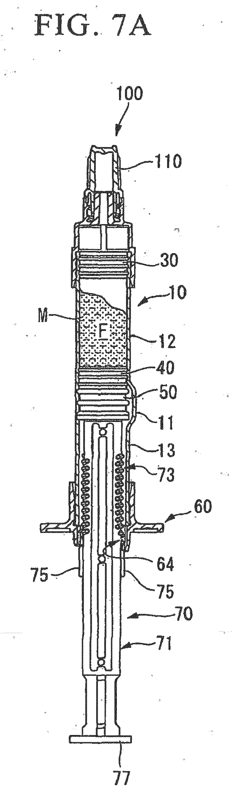

As shown in FIG 1, a dual chamber prefillable syringe (aripiprazole filled in a syringe) 100 is provided with a cylinder 10, a hub luer-lock 20, a front stopper 30, a middle stopper 40, an end stopper 50, a finger grip 60, and a plunger rod 70. In this dual chamber prefillable syringe 100, a preparation S and a solvent L to be mixed with each other to reconstitute injection M (see FIG. 7A) are filled in a separated state.

The dual chamber prefillable syringe 100 of this embodiment is suitable for use in injecting a material as the preparation S which is not easily dissolved in the solvent L, and even in this case, the preparation S can be easily and securely dissolved and suspended in the solvent L. Particularly, when aripiprazole is used as the preparation S which is not easily dissolved in the solvent L, the worth of the dual chamber prefillable syringe 100 of this embodiment is demonstrated.

The aripiprazole, that is,
7-{4-[4-(2,3-dichlorophenyl)-1-piperazinyl]butoxy}-3,4-dihydrocarbostyril, or 7-{4-[4-(2,3-dichlorophenyl)-1-piperazinyl]butoxy}-3,4-dihydro-2(1H)-quinolinone is atypical antipsychotic drug which is useful for treatment for schizophrenics, and has the following structural formula:

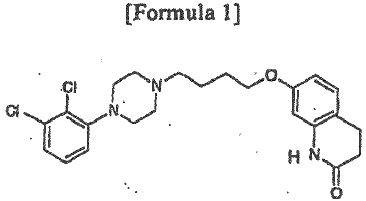

The cylinder 10 is molded from transparent glass, and has an approximately cylindrical shape extending along the axis line O. An approximately central part of the cylinder 10 in the direction of the axis line O is formed as a bypass portion 11 in which a part of the outer circumferential surface and the inner circumferential surface of the cylinder 10 in the circumferential direction bulges to the outside in the radial direction over a predetermined dimension in the direction of the axis line O. The position of the bypass portion 11 in the direction of the axis line O can be appropriately set in accordance with the design.

In addition, a cylindrical part of the cylinder 10 on the front end side of the bypass portion 11 is formed as a front-end-side cylinder portion 12, and a cylindrical part of the bypass portion 11 on the rear end side is formed as a rear-end-side cylinder portion 13. That is, the cylinder 10 has the front-end-side cylinder portion 12 which is disposed on the front end side with the bypass portion 11 as a boundary and the rear-end-side cylinder portion 13 which is disposed on the rear end side. That is, in the cylinder 10, an area on the front end side is formed as the front-end-side cylinder portion 12, an area on the rear end side is formed as the rear-end-side cylinder portion 13, and an area between the area on the front end side and the area on the rear end side is formed as the bypass portion 11.

In addition, a ring-shaped front-end-side protrusion 14 which protrudes to the outside in the radial direction over the entire area in the circumferential direction is formed at the outer circumference at the front end of the cylinder 10. Furthermore, a ring-shaped rear-end-side protrusion 15 which protrudes to the outside in the radial direction over the entire area in the circumferential direction is formed at the outer circumference at the rear end of the cylinder 10.

The hub luer-lock 20 is molded from a transparent synthetic resin having appropriate rigidity, and has a multi-stage cylindrical outer shape with the axis line O as a center. The hub luer-lock 20 is provided with a base end portion 21 having a cylindrical shape, a cylinder portion 22 which is coupled to the front end side of the base end portion 21 so as to be reduced by one stage in diameter, and a luer tip 23 which is formed on the front end side of the cylinder portion 22 to have a smaller diameter than the cylinder portion 22.

A fitting hole 24 which is opened to the rear end side of the hub luer-lock 20 is formed inside the base end portion 21, and a bypass chamber 25 having a shape of a hole with a bottom is formed on the front side of the fitting hole 24, that is, inside the cylinder portion 22. A front end surface 25a with which the front end of the front stopper 30 is brought into contact is formed at a position touching the bottom portion of the bypass chamber 25. The front end surface 25a is formed in a conical surface shape of which the diameter is gradually reduced toward the front side.

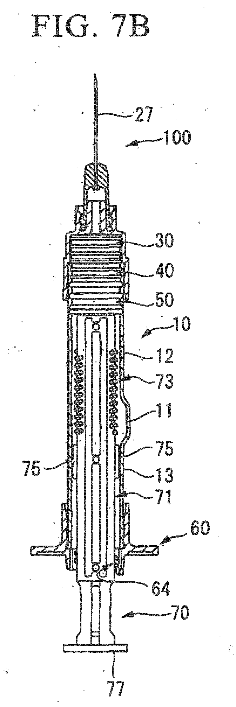

In addition, an introducing hole 23a penetrated along the axis line O is formed inside the luer tip 23. One end of the introducing hole 23a is opened to the front end of the luer tip 23, and the other end is opened to the center of the front end surface 25a of the bypass chamber 25. An injection needle 27 (omitted in FIG 1, see FIG 7B) extending to the front end side along the axis line O is attached to one end side, that is, the front end side of the introducing hole 23a so as to communicate therewith. A cap 110 is fitted to the luer tip 23.

The fitting hole 24 is a hole formed in order to attach the hub luer-lock 20 to the cylinder 10, and the inner diameter thereof is formed to be approximately the same as the outer diameter of the cylinder 10. The hub luer-lock 20 is attached to the front end side of the cylinder 10 by enclosing the front end of the cylinder 10 with the fitting hole 24.

In addition, a ring-shaped groove 24a recessed annularly is formed at the front end portion of the inner circumferential wall of the fitting hole 24 with the axis line O as a center. When the hub luer-lock 20 is attached to the front end side of the cylinder 10, the ring-shaped front-end-side protrusion 14 of the cylinder 10 is fitted to the ring-shaped groove 24a, and thus the hub luer-lock 20 is fixed integrally with the cylinder 10 air-tightly, liquid-tightly, and strongly.

The above-described bypass chamber 25 is a hole with a bottom, of which the inner diameter is smaller than the diameter of the fitting hole 24 by one stage, and the inner circumferential wall thereof has a bypass groove 26 formed thereon. The bypass groove 26 is constituted of a linear groove 26a and an annular groove 26b.

More than one linear grooves 26a are formed at regular intervals in the circumferential direction so as to extend parallel to the axis line O on the inner wall surface of the bypass chamber 25, and the front end sides of these linear grooves 26a are connected respectively to the introducing hole 23a extending to the front end surface 25a from the inner wall surface of the bypass chamber 25 and formed inside the luer tip 23.

In addition, the annular groove 26b is an annular groove extending in the circumferential direction with the axis line O with a center, and is formed in the vicinity of the boundary between the fitting hole 24 and the bypass chamber 25 on the inner wall surface of the bypass chamber 25. The annular groove 26b is connected to the respective rear ends of the above-described plurality of linear grooves 26a, and thus the respective linear grooves 26a are connected via the annular groove 26b.

The front stopper 30, the middle stopper 40, and the end stopper 50 are molded from medical rubber having corrosion resistance to the preparation S, the solvent L, and the injection M, and have an approximately cylindrical shape, which has an outer diameter slightly larger than the inner diameter of the cylinder 10, with the axis line O as a center.

The front stopper 30 is fitted to the front end side of the bypass portion 11 of the cylinder 10, that is, fitted in the front-end-side cylinder portion 12.

In addition, the middle stopper 40 is fitted to the rear end side of the bypass portion 11 of the cylinder 10, that is, fitted to the rear-end-side cylinder portion 13. Particularly, the middle stopper 40 of this embodiment is disposed so that the front end of the middle stopper 40 is positioned at the boundary between the bypass portion 11 and the rear-end-side cylinder portion 13 which is the front end of the rear-end-side cylinder portion 13. The powdery preparation S is sealed so as to be sandwiched between the middle stopper 40 and the front stopper 30 in the cylinder 10. That is, the preparation S is filled in a front chamber F formed by the inner circumferential surface of the cylinder 10, the rear end surface of the front stopper 30, and the front end surface of the middle stopper 40.

The end stopper 50 is fitted to the further rear end side of the middle stopper 40 of the rear-end-side cylinder portion 13 of the cylinder 10 while being spaced from the middle stopper 40 in the direction of the axis line O. The solvent L having a liquid form is sealed to be sandwiched between the end stopper 50 and the above-described middle stopper 40. That is, the solvent L is filled in a rear chamber B formed by the inner circumferential surface of the cylinder 10, the rear end surface of the middle stopper 40, and the front end surface of the end stopper 50. A female screw hole (omitted) into which a connecting portion 76 of the plunger rod 70 to be described later is screwed is formed at the rear end of the end stopper 50.

In this manner, in the dual chamber prefillable syringe 100, the preparation S and the solvent L are separately sealed in the front chamber F and the rear chamber B divided by the middle stopper 40.

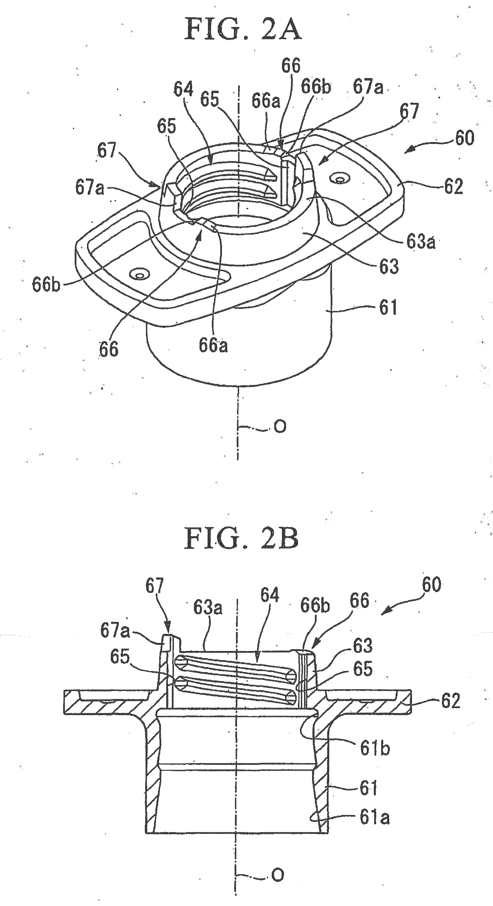

As shown in FIGS. 2A and 2B, the finger grip 60 is provided with a fitting portion 61, a flange portion 62, and a cylinder portion 63.

The fitting portion 61 has an approximately cylindrical shape with the axis line O as a center, and a fitting hole 61a to which the rear end of the cylinder 10 is fitted is formed on the inner circumferential side thereof. A ring-shaped groove 61b recessed annularly is formed at the inner circumference at the rear end of the fitting hole 61 a with the axis line O as a center. As shown in FIG. 1, when the finger grip 60 is attached to the rear end of the cylinder 10, the ring-shaped rear-end-side protrusion 15 of the cylinder 10 is fitted to the ring-shaped groove 61b, and thus the finger grip 60 is strongly fixed integrally with the cylinder 10.

The flange portion 62 projects in the diametrical direction from the rear end of the fitting portion 61, that is, from the vicinity of the boundary between the fitting portion 61 and the cylinder portion 63 with the axis line O as a center, and has an approximately rectangular shape when viewed in the direction of the axis line O. The flange portion 62 supports fingers of a healthcare worker when the dual chamber prefillable syringe 100 is used, and thus acts to make the handling of the dual chamber prefillable syringe 100 by the healthcare worker easy.

The cylinder portion 63 has an approximately cylindrical shape with the axis line O as a center, and further extends toward the rear side from the rear end of the fitting portion 61. The cylinder portion 63 has an inner diameter which is smaller than the inner diameter of the fitting portion 61 by one stage, and a step portion at the boundary between the cylinder portion 63 and the fitting portion 61 is brought into contact with the rear end of the cylinder 10. A female screw portion 64 which is screwed around the axis line O is formed on the inner circumferential surface of the cylinder portion 63. The female screw portion 64 is constituted of two female screws which are screwed in the clockwise direction (hereinafter, referred to as the screw rotation direction) toward the front end side from the rear end side of the finger grip 60, and each female screw extends to turn around over, for example, 360° of the inner circumferential surface of the cylinder portion 63.

In addition, a guide groove 65 which is recessed toward the outside (outside in the radial direction of the axis line O) in the radial direction of the female screw portion 64 and extends parallel to the axis line O is formed on the inner circumferential surface of the cylinder portion 63, that is, in the female screw portion 64. The guide groove 65 extends over the entire areas of the cylinder portion 63 and the female screw portion 64 in the direction of the axis line O, and a pair of the guide grooves 65 are provided to be opposed to each other with an interval of 180° therebetween in the circumferential direction of the female screw portion 64, that is, opposed to each other in the diametrical direction of the female screw portion 64.

In addition, a pair of first protrusions 66 and a pair of second protrusions 67 are provided at the rear end of the cylinder portion 63. The pair of first protrusions 66 are provided to be opposed to each other with an interval of 180° therebetween in the circumferential direction of the cylinder portion, that is, opposed to each other in the diametrical direction of the cylinder portion. In addition, the pair of second protrusions 67 are also provided to be opposed to each other with an interval of 180° therebetween in the circumferential direction of the cylinder portion, that is, opposed to each other in the diametrical direction of the cylinder portion.

The first protrusion 66 is formed at a part on the rear side of the pair of guide grooves 65 of the rear end surface 63a of the cylinder portion 63 in the screw rotation direction to protrude to the rear side along the axis line O from the rear end surface 63a. In addition, the second protrusion 67 is formed at a part on the front side of the pair of guide grooves 65 of the rear end surface 63a of the cylinder portion 63 in the screw rotation direction to protrude to the rear side along the axis line O from the rear end surface 63a. That is, the first and second protrusions 66 and 67 are adjacent to the guide grooves 65 to sandwich the guide grooves 65 therebetween in the circumferential direction of the axis line O.

Here, a surface of the first protrusion 66 which faces the rear side in the screw rotation direction is formed as a gently-inclined surface 66a which is gradually inclined toward the rear side along the axis line O from the rear end surface 63a facing the front side in the screw rotation direction. In addition, a surface facing the front side in the screw rotation direction of the first protrusion 66 is formed as a steeply-inclined surface 66b which is gradually inclined toward the front side along the axis line O facing the front side in the screw rotation direction. The steeply-inclined surface 66b has a steeper inclination than the above-described gently-inclined surface 66a.

Furthermore, a surface of the second protrusion 67 which faces the rear side in the screw rotation direction is formed as a vertical surface 67a which vertically extends to the rear side along the axis line O from the rear end surface 63a of the cylinder portion 63 connected to the guide grooves 65.

The second protrusion 67 is formed to be higher than the first protrusion 66, that is, the top portion of the vertical surface 67a of the second protrusion 67 is positioned closer to the rear side in the direction of the axis line O than the top portion of the first protrusion 66.

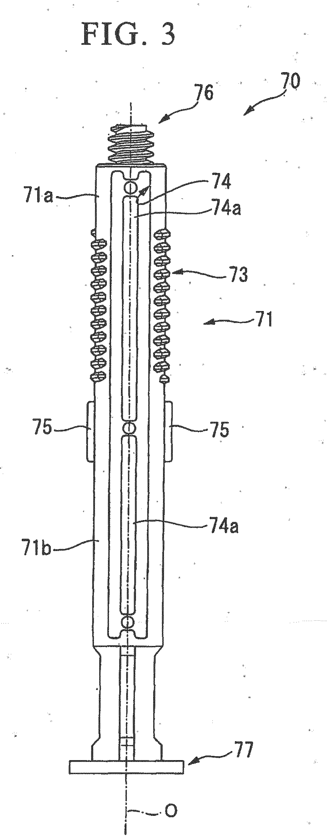

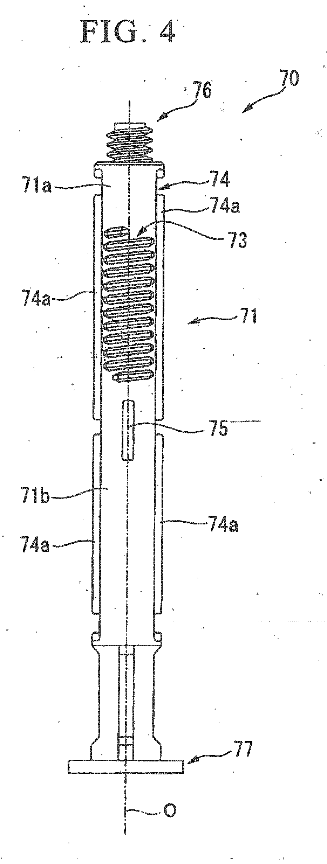

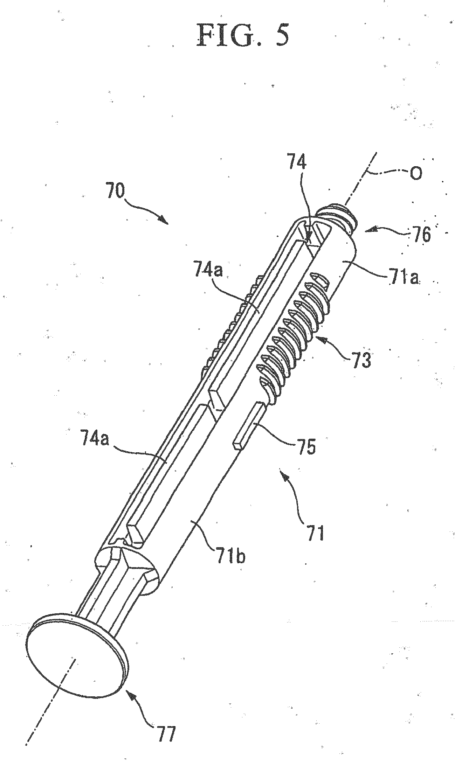

The plunger rod 70 is a member connected to the end stopper 50 to move the end stopper 50 to the front side of the cylinder 10. As show in FIGS. 3 to 5, the plunger rod 70 is provided with a long rod portion 71 extending along the axis line O, a connecting portion 76 which is provided on the front end side of the rod portion 71 and has a male screw shape connected to the end stopper 50, and a pressing portion 77 which is provided on the rear end side of the rod portion 71 and by which the healthcare worker applies pressure when pressing the end stopper 50.

In addition, a male screw portion 73 which is screwed around the axis line O is formed on the outer circumferential surface of the rod portion 71. The male screw portion 73 is constituted of two male screws which are screwed in the clockwise direction (screw rotation direction) toward the front end side from the rear end side of the plunger rod 70. Such a male screw portion 73 is formed over a predetermined range in the direction of the axis line O more on the front end side than a central portion of the rod portion 71 in the direction of the axis line O.

The rod portion 71 has a pair of cutout portions 74 which are formed by cutting out a predetermined range in the circumferential direction over substantially the entire area in the direction of the axis line O. The pair of cutout portions 74 are formed with an interval of 180° therebetween in the circumferential direction of the rod portion 71. Accordingly, the rod portion 71 can be molded using a split mold.

By the cutout portions 74, the male screw portion 73 is divided into two parts in the circumferential direction of the rod portion 71.

In the cutout portion 74, a rib 74a is formed which protrudes to the outside in the radial direction of the axis line O from a bottom surface of the cutout portion 74, that is, from a surface of the cutout portion 74 facing the outside in the radial direction of the axis line O to extend parallel to the axis line O. The strength of the rod portion 71 is held by the rib 74. In addition, two ribs 74a are arranged in parallel in the direction of the axis line O in the respective cutout portions 74. Due to the configuration in which the rib 74a on the front end side and the rib 74a on the rear end side are divided in this manner, a part of the solvent L remaining in the bulging part of the bypass portion 11 can be avoided from reaching the rib 74a on the rear end side through the rib 74a on the front end side and adhering to a hand of the healthcare worker.

In addition, an area on the front end side of the area in which the male screw portion 73 of the rod portion 71 is formed is formed as a rod front-end portion 71 a having a cylindrical outer circumferential surface, and an area on the rear end side of the area in which the male screw portion 73 is formed is formed as a rod rear-end portion 71b having the same cylindrical outer circumferential surface.

The rod front-end portion 71a and the rod rear-end portion 71b are formed to have an outer diameter which is the same or slightly smaller than the inner diameter of the female screw portion 64 of the cylinder portion 63 of the finger grip 60. In this manner, the rod front-end portion 71 a and the rod rear-end portion 71b can be inserted into the female screw portion 64 in the direction of the axis line O.

Furthermore, a pair of guide plates 75 are formed with an interval of 180° therebetween in the circumferential direction of the rod portion 71 on the outer circumferential surface of the rod rear-end portion 71b. These guide plates 75 have a shape rectangularly protruding to the outside in the radial direction of the rod portion 71 in a cross-section perpendicular to the axis line O, and extend parallel to the axis line O over a predetermined range.

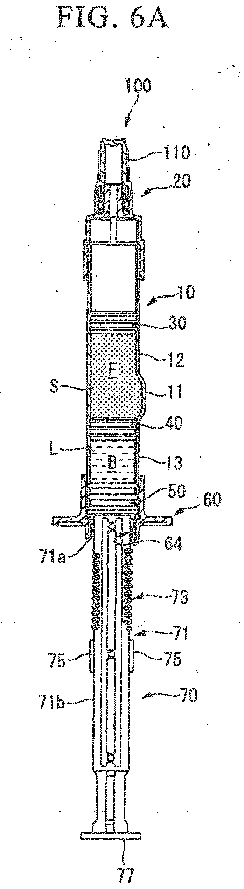

Next, a method of using the dual chamber prefillable syringe 100 having the above-described configuration will be described with reference to FIGS. 6A to 7B.

First, as shown in FIG. 6A, the connecting portion 76 at the front end of the plunger rod 70 is screwed into a female screw hole of the end stopper 50, and thus the plunger rod 70 is connected to the end stopper 50. In this state, the rod front-end portion 71a of the rod portion 71 is inserted into the female screw portion 64 of the finger grip 60.

Next, the pressing portion 77 of the plunger rod 70 is pressed from the rear end side in a state in which fingers of the healthcare worker are put on the flange portion 62 of the finger grip 60. The pressing force is transmitted to the middle stopper 40 via the end stopper 50 and the solvent L. That is, the plunger rod 70 advances due to the above-described pressing force, and the middle stopper 40 also advances in the rear-end-side cylinder portion 13 of the cylinder 10.

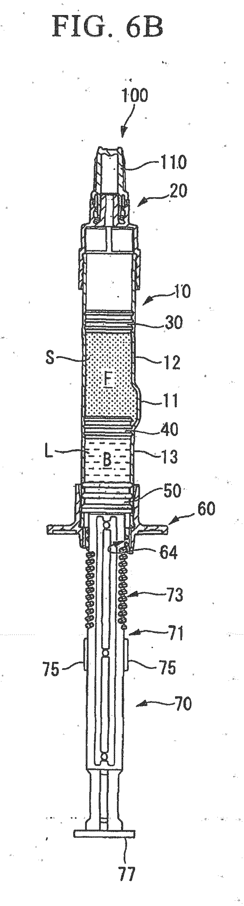

As shown in FIG. 6B, at a point of time when a part of the middle stopper 40 (in this embodiment, half of the middle stopper 40 on the front end side in the direction of the axis line O) enters into the bypass portion 11, the front end of the male screw portion 73 of the plunger rod 70 is brought into contact with the rear end of the female screw portion 64 of the finger grip 60. The male screw portion 73 may be brought into contact with the female screw portion 64 when the front end of the middle stopper 40 reaches the rear end of the bypass portion 11, or the male screw portion 73 may reach the female screw portion 64 immediately before the front end of the middle stopper 40 reaches the rear end of the bypass portion 11. By bringing the male screw portion 73 and the female screw portion 64 into contact with each other in this manner, the male screw portion 73 and the female screw portion 64 become stoppers of the advance of the plunger rod 70. Afterward, the plunger stopper 70 cannot be advanced even when the plunger rod 70 is pressed.

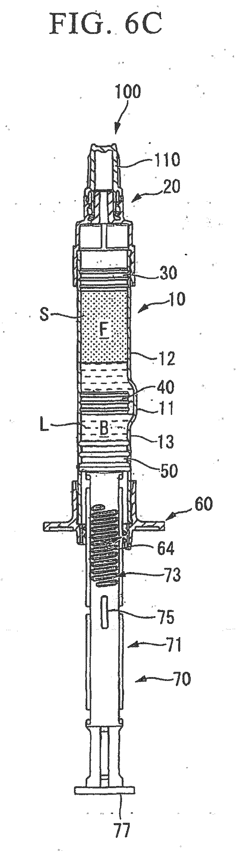

In addition, by bringing the male screw portion 73 and the female screw portion 64 into contact with each other in this manner, the male screw portion 73 and the female screw portion 64 can be screwed together. Accordingly, when the plunger rod 70 is rotated to the front side in the screw rotation direction in this state, the male screw portion 73 and the female screw portion 64 are screwed together, and the plunger rod 70 advances in accordance with the pitches thereof. The end stopper 50 also advances with the advance of the plunger rod 70. As shown in FIG. 6C, when the entire length of the middle stopper 40 in the direction of the axis lone O enters into the inside of the bypass portion 11, the front chamber F and the rear chamber B communicate with each other via the outwardly bulging area of the cylinder 10 in the bypass portion 11.

Accordingly, the solvent L of the rear chamber B can be allowed to flow to the preparation S of the front chamber F. Moreover, when the plunger rod 70 is further rotated in the screw rotation direction and gradually advanced, most of the pressing force which is applied to the solvent L due to the advance of the plunger rod 70 is converted into pressure for allowing the solvent L to flow to the front chamber F. Therefore, the middle stopper 40 stays in the bypass portion 11 with little advance.

Thereafter, at a point of time when the front end of the end stopper 50 is brought into contact with the rear end of the middle stopper 40 due to the advance of the plunger rod 70, the entire solvent L in the rear chamber B is introduced to the preparation S of the front chamber F, and thus the rear chamber B is eliminated. Moreover, when the plunger rod 70 is further rotated in the screw rotation direction and gradually advanced, the middle stopper 40 which is brought into contact with the end stopper 50 is also simultaneously advanced via the end stopper 50.

Next, as shown in FIG. 7A, at a point of time when a part of the middle stopper 40 (in this embodiment, half of the middle stopper 40 on the front end side in the direction of the axis line O) enters into the front-end-side cylinder portion 12 of the cylinder 10, that is, at a point of time when a part of the middle stopper 40 moves out of the bypass portion 11, the guide plate 75 of the plunger rod 70 is brought into contact with the gently-inclined surface 66a of the first protrusion 66 of the finger grip 60 so as to be able to surmount thereover from the rear side in the screw rotation direction. At this time, the front chamber F is sealed again and the healthcare worker shakes the dual chamber prefillable syringe 100 under this circumstance, so that the preparation S is completely dissolved by the solvent L and the preparation of the injection M is completed.

Here, the state in which the guide plate 75 is brought into contact with the first protrusion 66 so as to be able to surmount thereover means a contact state at a level where even though a resistance occurs in the rotation of the plunger rod 70 due to the guide plate 75 brought into contact with the gently-inclined surface 66a of the first protrusion 66, the guide plate 75 can surmount the first protrusion 66 to the front side in the screw rotation direction by strongly rotating the plunger rod 70. Such a contact state can be realized by molding the plunger rod 70 and the finger grip 60 with a flexible material such as a synthetic resin.

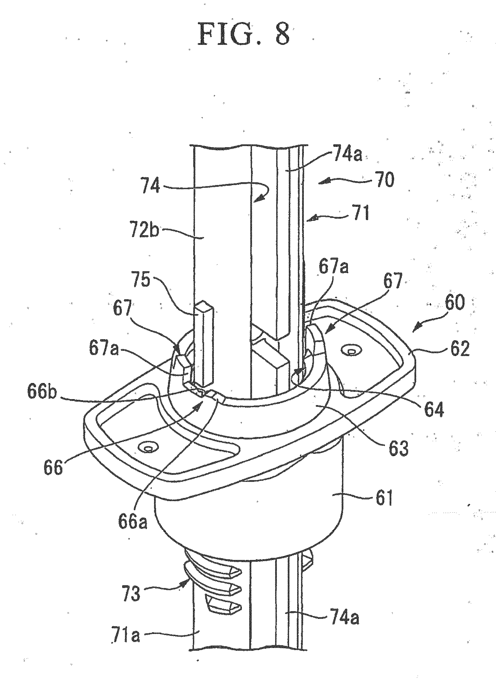

In the above-described contact state, when the guide plate 75 surmounts the first protrusion 66 as shown in FIG. 8 by strongly rotating the plunger rod 70, the guide plate 75 is then brought into contact with the vertical surface 67a of the second protrusion 67 from the rear side in the screw rotation direction. Accordingly, the movement of the guide plate 75 to the front side in the screw rotation direction is inhibited. That is, the plunger rod 70 cannot be rotated to the front side in the screw rotation direction anymore. Even when the plunger rod 70 is rotated in the inverse screw rotation direction, the guide plate 75 is brought into contact with the steeply-inclined surface 66a of the first protrusion 66, and thus the rotation is inhibited.

Furthermore, after the guide plate 75 surmounts the first protrusion 66 as described above, the male screw portion 73 passes through an area in which the female screw portion 64 is present. That is, the screwing between the male screw portion 73 and the female screw portion 64 is released. In addition, when the guide plate 75 is positioned between the first and second protrusions 66 and 67, the position of the guide plate 75 in the circumferential direction coincides with that of the guide groove 65 of the finger grip 60. That is, the guide plate 75 and the guide groove 65 can be fitted to each other.

Accordingly, the plunger rod 70 can be pressed once again to go straight.

Thereafter, when the plunger rod 70 is pressed, the guide plate 75 of the plunger rod 70 is fitted to the guide groove 65 of the finger grip 60, and the plunger rod 70 advances to be guided to the guide groove 65. The pressing force which is generated by pressing the plunger rod 70 at this time is transmitted to the front stopper 30 via the end stopper 50, the middle stopper 40, and the injection M, and the front stopper 30 advances in the cylinder 10.

When the front stopper 30 enters the bypass chamber 25 as a result of the advance of the above-described front stopper 30, the front chamber F in which the injection M is present communicates with the introducing hole 23a of the luer tip 23 via the bypass groove 26. Accordingly, bubbles remaining in the cylinder 10 are discharged to the outside, and thus the injection M can be introduced to the injection needle 27, and the injection M can be injected to a patient.

Thereafter, in the injection of the injection M, the plunger rod 70 is further pressed, and thus the injection M of the front chamber F is introduced to the injection needle 27 via the bypass groove 26 and the introducing hole 23a. Moreover, when the plunger rod 70 is completely pressed, as shown in FIG. 7B, the entire injection M is injected to a patient via the injection needle 27, and the front end of the middle stopper 40 and the rear end of the front stopper 30 are brought into contact with each other, whereby the front chamber F is eliminated. As described above, the injection of the injection M to a patient ends.

According to the dual chamber prefillable syringe 100 of this embodiment having the above-described configuration, when the plunger rod 70 is rotated in accordance with the screwing between the male screw portion 73 and the female screw portion 64, the plunger rod 70 gradually advances in accordance with the pitches of the male screw portion 73 and the female screw portion 64. Accordingly, it is possible to avoid the excessive advancing speed of the plunger rod 70, and it is possible to allow the middle stopper 40 to stay in the bypass portion 11. Accordingly, it is possible to secure the communication state between the front chamber F and the rear chamber B, and thus it is possible to appropriately (or completely) mix the solvent L and the preparation S.

Here, when aripiprazole is employed as the preparation S, it is difficult to dissolve in the solvent L. However, in the dual chamber prefillable syringe 100 of this embodiment, the preparation S can be appropriately (or completely) dissolved in the solvent L. Therefore, even when aripiprazole is employed as the preparation S which is difficult to dissolve in the solvent L, the aripiprazole can be easily suspended in the solvent L.

In addition, when the middle stopper 40 is advanced via the end stopper 50 and the solvent L by pressing the plunger rod 70, the male screw portion 73 is brought into contact with the female screw portion 64 when a part of the middle stopper 40 enters into the bypass portion 11. Accordingly, even when the plunger rod 70 is further pressed, the plunger rod 70 does not advance, and the middle stopper 40 also does not advance. Therefore, it is possible to avoid the middle stopper 40 being excessively advanced up to the front end side of the bypass portion 11 due to an excessive force applied to the plunge rod 70.

In addition, the dual chamber prefillable syringe 100 of this embodiment has a configuration in which as described above, when a part of the middle stopper 40 advancing by pressing the plunger rod 70 enters into the bypass portion 11, the front end of the male screw portion 73 reaches and is screwed to the front end of the female screw portion 64. A configuration may also be provided in which the front end of the male screw portion 73 reaches the front end of the female screw portion 64 immediately before the front end of the middle stopper 40 enters into the bypass portion 11.

Accordingly, since the plunger rod 70 can be rotated in accordance with the screwing between the male screw portion 73 and the female screw portion 64 only when the middle stopper 40 reaches the bypass portion 11 and the solvent L is introduced to the preparation S, only an operation of pressing the plunger rod 70 may be performed until a part of the middle stopper 40 reaches the bypass portion 11. Thus, it is possible to simplify the handling of the dual chamber prefillable syringe 100.

Furthermore, in the dual chamber prefillable syringe 100 according to this embodiment, when the plunger rod 70 is rotated and advanced in accordance with the screwing between the male screw portion 73 and the female screw portion 64, the male screw portion 73 of the plunger rod 70 passes through the female screw portion 64 of the finger grip 60, and thus the screwing between the male screw portion 73 and the female screw portion 64 is released. At this time, the guide plate 75 of the plunger rod 70 is brought into contact with the second protrusion 67 of the finger grip 60, and the plunger rod 70 cannot be rotated anymore. Therefore, the healthcare worker can easily recognize that the screwing between the male screw portion 73 and the female screw portion 64 has been released.

In addition, since the screwing is released in this manner, the plunger rod 70 can be pressed. At this time, the plunger rod 70 is stopped at a position in which the guide plate 75 of the plunger rod 70 and the guide groove 65 formed in the female screw portion 64 can be fitted to each other, and thus when the plunger rod 70 is pressed, the plunger rod 70 can be guided in the direction in which the guide groove 65 extends. Accordingly, the plunger rod 70 can be allowed to go straight, and the same operation as in the case of normal syringes can be performed.

In addition, in this embodiment, in the case in which the plunger rod 70 is rotated in accordance with the screwing between the male screw portion 73 and the female screw portion 64, the guide plate 75 of the plunger rod 70 is brought into contact with the first protrusion 66 so as to surmount thereover when the middle stopper 40 going straight in the cylinder 10 moves out of the bypass portion 11. Accordingly, the healthcare worker can easily recognize that the front chamber F is sealed again since a part of the middle stopper 40 moves across the bypass portion 11. At this time, the healthcare worker shakes the dual chamber prefillable syringe 100, so that the injection M in which the preparation S is completely dissolved in the solvent L can be reconstituted.

Furthermore, in this embodiment, as described above, the configuration is provided in which the guide plate 75 surmounts the first protrusion 66 by rotating the plunger rod 70 in accordance with the screwing between the female screw portion 64 and the male screw portion 73, and then the screwing between the male screw portion 73 and the female screw portion 64 is released.

Accordingly, after a part of the middle stopper 40 moves across the bypass portion 11 due to the rotation of the plunger rod 70, the operation of pressing the plunger rod 70 can be performed. In addition, after the guide plate 75 surmounts the first protrusion 66, the guide plate 75 is brought into contact with the second protrusion 67, and thus the rotation of the plunger rod 70 is stopped. This position becomes a position in which the advance due to the rotation and the advance due to the going straight are switched.

In this manner, the plunger rod 70 is rotated only when introducing the solvent L to the preparation S, and then the operation can be switched to an operation of pressing the plunger rod 70. It is possible to simplify the handling of the dual chamber prefillable syringe 100.

In addition, in this embodiment, when the guide plate 75 is brought into contact with the second protrusion 67 by rotating the plunger rod 70, the guide plate 75 of the plunger rod 70 is fitted to the guide groove 65 of the finger grip 60. Accordingly, thereafter, by pressing the plunger rod 70, the plunger rod 70 can be allowed to go straight.

As described above, the dual chamber prefillable syringe 100 of this embodiment is used to allow the plunger rod 70 to go straight when used to thereby allow the middle stopper 40 to enter into the bypass portion 11, thereby allowing the solvent L to securely flow to the preparation S and sufficiently dissolving or suspending the preparation S. Here, when the plunger rod 70 goes straight only, the force to press the plunger rod 70 is difficult to adjust, and thus there is a possibility that the middle stopper 40 may pass through the bypass portion 11.

In this embodiment, by the contact of the male screw portion 73 with the female screw portion 64, the healthcare worker can detect a position in which the middle stopper 40 enters into the bypass portion 11. In addition, the subsequent advance of the plunger rod 70 is performed by the rotation according to the screwing between the male screw portion 73 and the female screw portion 64, and thus the speed can be easily adjusted and the advancing speed does not increase to a predetermined speed or higher. Accordingly, it is possible to avoid the middle stopper 40 passing through the bypass portion 11. Particularly, the dual chamber prefillable syringe 100 of this embodiment is suitable to be applied when the preparation S is special and it takes a long time to dissolve the preparation S.

In addition, it is considered that if the guide plate 75 and the second protrusion 67 are not present when the male screw portion 73 passes through the female screw portion 64 and the fastening between the male screw portion 73 and the female screw portion 64 ends, the healthcare worker cannot detect the release of the screwing between the male screw portion 73 and the female screw portion 64, and at that position, the plunger rod 70 is rotated again and again. In addition, when the healthcare worker notices the release of the above-described screwing and presses the plunger rod 70 in a hurry, the injection M is pressed instantaneously since the plunger rod 70 can be allowed to freely go straight, and thus the front stopper is dropped into the bypass chamber 25 and the sealing of the front end portion of the cylinder 10 is released. Whereby, when the syringe is shaken, there is concern that the solvent L or the injection M may fly out of the injection needle 27.

Regarding this, in this embodiment, the guide plate 75 is brought into contact with the second protrusion 67 to stop the rotation of the plunger rod 70, and thus it is possible to allow the healthcare worker to detect the release of the above-described screwing. Accordingly, the healthcare worker can recognize that it is necessary not to press the plunger rod 70 without careful consideration, it is necessary to sufficiently dissolve and suspend the preparation S by shaking the preparation S and the solvent L well, and it is necessary to operate the plunger rod 70 carefully to discharge bubbles from that position and inject the drug into a patient.

The dual chamber prefillable syringe (aripiprazole filled in a syringe) 100 which is an embodiment of the invention has been described in detail. However, the claimed invention is not limited thereto, and some modifications in design and the like can be made to the embodiments without departing from the scope of the claimed invention.

For example, the pitches of the male screw portion 73 and the female screw portion 64 can be appropriately designed, and thus the advancing speed of the plunger rod 70 can be adjusted, and the speed of the dissolution or suspending of the preparation S by the solvent L can be freely adjusted.

Moreover, in the case in which the dual chamber prefillable syringe 100 is packaged in a state in which the plunger rod 70 is connected to the end stopper 50, even when the plunger rod 70 is pressed carelessly during the transport and the like, the further advance of the plunger rod 70 is inhibited since the male screw portion 73 is brought into contact with the female screw portion 64. Accordingly, it is possible to always securely hold the separation state of the solvent L and the preparation S, and it is possible to avoid the danger.

It is preferable that the respective end portions of the female screw portion 64 and the male screw portion 73 in the circumferential direction be formed in a tapered shape. Accordingly, the female screw portion 64 and the male screw portion 73 can be easily screwed to each other, and thus the operation can be smoothly performed.

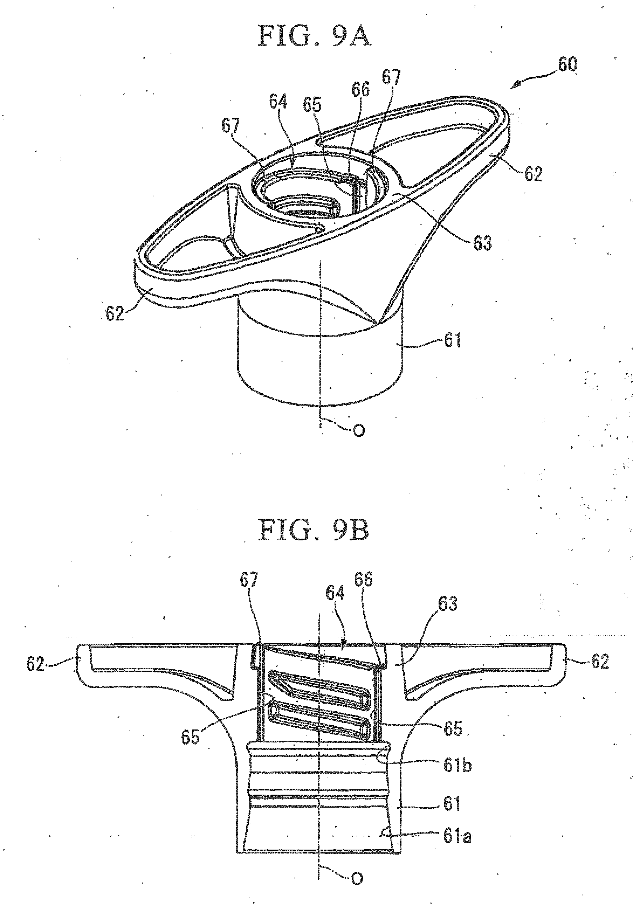

It is possible to use a finger grip 60 as shown in FIGS. 9A and 9B, for example, as the finger grip 60 of a modified embodiment. In this finger grip 60 of the other embodiment, the flange portion 62 is provided at the rear end side of the cylinder portion 63, with different to the embodiment in which the cylinder portion 63 is rearwardly projecting from the flange portion 62. In other words, in case of the modified embodiment, the cylinder portion 63 is buried in the fitting portion 61.

According to this, since the cylinder portion 63 having the female screw portion 64, the first protrusions 66, the second protrusions 67 and the guide groove 65 does not projecting from the flange portion 62, it is possible to avoid the handling of the dual chamber prefillable syringe from becoming troublesome due to the fingers of the healthcare worker touch the cylinder portion 63.

According to the dual chamber prefillable syringe of embodiments of the invention, it is possible to appropriately mix the solvent and the preparation. In addition, according to the aripiprazole filled in a syringe, it is possible to reduce the residue and incomplete dissolution in resuspending.

### Examples

Hereinafter, examples will be described.

An aripiprazole hydrate bulk powder was suspended in a dispersion medium to obtain a concentration of 30 wt% as aripiprazole anhydride. The concentrations of other additives in the obtained suspension were as follows. The concentration of carboxymethylcellulose sodium was about 1.248 wt%, the concentration of mannitol was about 6.24 wt%, and the concentration of sodium dihydrogenphosphate monohydrate was 0.111 wt%. In addition, pH of the suspension was adjusted to about 7 by adding aqueous sodium hydroxide. The suspension was wet -milled by a high shear rotary homogenizer (CLEARMIX, M Technique Co., Ltd.) and repeatedly wet -milled at 550 bar by a high-pressure homogenizer (Niro Inc.). The primary mean particle size of the obtained suspension was 2 to 3 µm.

1.5 mL of the suspension (about 450 mg, as aripiprazole anhydride) was filled in a cup for freeze-drying made of polyethylene, and moved to a freeze dryer. The suspension was freeze-dried in accordance with the following cycle of (a) and (b) to obtain a cake composition:
(a) Heat Treatment: The cup filled with the suspension was frozen for about 4 hours at a shelf temperature maintained to about -40°C; and
(b) Primary Drying: Primary drying was continued for about 40 hours at a chamber pressure increased to about 13 Pa and a shelf temperature increased to about -5°C

After freeze-drying, the bottom of the cup was pressed and a firm freeze-dried cake was taken.

As a preparation S, the freeze-dried cake was put in the front chamber (space between the front stopper 30 and the middle stopper 40) of the dual chamber prefillable syringe 100 of the embodiment. The rear chamber (space between the middle stopper 40 and the end stopper 50) was filled with 2.0 mL of purified water as a solvent L.

### 1. Measurement of Remaining Amount in Dead Space in Dual chamber Prefillable syringe

The end stopper 50 was pressed until the purified water of the rear chamber flowed to the front chamber via the bypass 11. After flowing of the purified water to the front chamber, the freeze-dried cake was resuspended through sufficient shaking. In the suspension after resuspending, lumps of powder and the like due to the residue caused by incomplete dissolution were not shown. The suspension was discharged from the introducing hole 23a of the luer tip 23, which was the discharge port at the front end of the dual chamber prefillable syringe 100, by further pressing the end stopper 50. After discharge, the dual chamber prefillable syringe 100 was disassembled, and the amount of the drug remaining in the dual chamber prefillable syringe 100 was quantitated. The average thereof was about 19 mg. Since the suspension was shaken well and uniformly resuspended, the amount of the drug is a remaining amount in the dead space in the syringe.

### 2. Resuspending in General Plunger Rod

A general plunger rod (with no male screw portion 73 formed therein) only for unidirectional pressing in the discharge direction was attached to the end stopper 50 to make a dual chamber prefillable syringe of a comparative example, and the end stopper 50 was pressed to allow purified water to rapidly flow to the front chamber in the same manner as described above. Then, the syringe was left for 5 seconds without complete shaking, and the suspension was discharged from the introducing hole 23a of the luer tip 23. After discharge, the dual chamber prefillable syringe was disassembled, and the amount of the drug remaining in the dual chamber prefillable syringe was quantitated. The average thereof was about 195 mg. It was found that about 176 mg, which is a difference with the above-described remaining amount, remains in the syringe as the residue caused by incomplete dissolution.

### 3. Resuspending in Screw-Type Plunger Rod

The screw-type plunger rod 70 of this embodiment was attached to the end stopper 50 to allow purified water to rapidly flow to the front chamber while turning the screw as described above in this embodiment. Then, the syringe was left for 5 seconds without complete shaking, and the suspension was discharged from the introducing hole 23a of the luer tip 23. After discharge, the dual chamber prefillable syringe was disassembled, and the amount of the drug remaining in the dual chamber prefillable syringe was quantitated. The average thereof was about 62 mg. It was found that about 43 mg, which is a difference with the above-described remaining amount, remains in the syringe as the residue caused by incomplete dissolution.

The result of the above-described test is shown in Table 1. From the result, it was found that the resuspending can be efficiently performed by performing a resuspending process with the screw-type plunger rod 70 of this embodiment. In general, in the dual chamber prefillable syringe, there is concern that when resuspending by shaking is needed as in the case of the preparation used in this examination, the injection may be carried out without a shaking process in actual clinical practice. When the shaking process is forgotten, a significant reduction in the dose is shown when using a general plunger rod. However, by using the plunger rod 70 of this embodiment, the significant reduction in the dose becomes smaller even when the shaking is forgotten. In addition, since the plunger rod 70 is operated differently before and after the resuspending process unlike the case of a general plunger rod only for pressing, it is possible to prompt the healthcare worker to recognize the resuspending process, and it is possible to reduce a risk of the reduction in the dose due to forgetting of the shaking.

**[Table 1]**

| | | Remaining Amount in Syringe (mg) (Aripiprazole anhydride amount) | Average (mg) |
|---|---|---|---|
| 1. Remaining Amount in Dead Space (with shaking) | n=1 | 23.8 | 18.5 |
| | n=2 | 15.0 | |
| | n=3 | 16.6 | |
| 2. Resuspending in General Plunger Rod (without shaking) | n=1 | 138.1 | 194.5 |
| | n=2 | 232.7 | |
| | n=3 | 212.7 | |
| 3. Resuspending in Screw-Type Plunger Rod (without shaking) | n=1 | 60.7 | 62.0 |
| | n=2 | 53.2 | |
| | n=3 | 72.0 | |

### Reference Signs List

10 cylinder, 11 bypass portion, 12 front-end-side cylinder portion, 13 rear-end-side cylinder portion, 14 ring-shaped front-end-side protrusion, 15 ring-shaped rear-end-side protrusion, 20 hub luer-lock, 21 base end portion, 22 cylinder portion, 23 luer tip, 24 fitting hole, 25 bypass chamber, 25a front end surface, 26 bypass groove, 26a linear groove, 26b annular groove, 27 needle, 30 front stopper, 40 middle stopper, 50 end stopper, 60 finger grip, 61 fitting portion, 62 flange portion, 63 cylinder portion, 64 female screw portion, 65 guide groove, 66 first protrusion, 66a gently-inclined surface, 66b steeply-inclined surface, 67 second protrusion, 67a vertical surface, 70 plunger rod, 71 rod portion, 71a rod front-end portion, 41b rod rear-end portion, 73 male screw portion, 74 cutout portion, 74a rib, 75 guide plate, 76 connecting portion, 77 pressing portion, 100 dual chamber prefillable syringe, O axis line, S preparation, L solvent, M injection, F front chamber, B rear chamber

## Claims

1. A dual chamber prefillable syringe (100), comprising:
a cylinder (10) which has a cylindrical shape with an axis line (O) as a center and has a bypass portion (11) formed due to bulging of a part of an inner circumferential surface to the outside;
a hub luer-lock (20) which is provided at the front end of the cylinder;
a finger grip (60) which is provided at the rear end of the cylinder;
a front stopper (30) which is fitted on the front end side of the bypass portion in the cylinder;
a middle stopper (40) which is fitted on the rear end side of the bypass portion in the cylinder to seal a preparation (S) together with the front stopper;
an end stopper (50) which is fitted on the rear end side of the middle stopper in the cylinder to seal a solvent together with the middle stopper;
a plunger rod (70) which is connected to the end stopper from the rear end side by inserting the finger grip and which is provided with a long rod portion extending along the axis line, a connecting portion which is provided on the front end side of the rod portion and has a male screw shape connected to the end stopper, and a pressing portion which is provided on the rear end side of the rod portion;
a female screw portion (64) which is formed to be screwed around the axis line on an inner circumferential surface of the finger grip; and
a male screw portion (73) which is formed over a predetermined range in the direction of the axis line more on the front end side than a central portion of the rod portion in the direction of the axis line and to be able to screw together with the female screw portion on an outer circumferential surface of the plunger rod,
**characterized by** a guide groove (65) which is formed in the female screw portion and extends parallel to the axis line;
a guide plate (75) which is formed on the rear end side of the male screw portion on the outer circumferential surface of the plunger rod and is guided in accordance with the guide groove; and
a first protrusion (66) which is formed at the rear end of the finger grip and with which the guide plate is arranged to be brought into contact, so as to be able to surmount thereover, when at least a part of the middle stopper, which is advanceable by rotating the plunger rod in accordance with the female screw portion and the male screw portion, moves across the bypass portion.

2. The dual chamber prefillable syringe according to Claim 1, wherein the front end of the male screw portion is arranged to reach the rear end of the female screw portion, and can be screwed thereto, when the middle stopper, which is advanced by pressing the plunger rod, enters into the bypass portion.

3. The dual chamber prefillable syringe according to Claim 1 or Claim 2, wherein the screwing between the male screw portion and the female screw portion is arranged to be released after the guide plate surmounts the first protrusion, by rotating the plunger rod in accordance with the female screw portion and the male screw portion.

4. The dual chamber prefillable syringe according to Claim 3, further comprising:
a second protrusion (67) which is formed at the rear end of the finger grip and is arranged to be brought into contact at a position in which the guide plate surmounting over the first protrusion can be fitted to the guide groove.

5. The dual chamber prefillable syringe according to Claims 1 to 4, wherein the preparation includes aripiprazole.

## Patentansprüche

1. Vorfüllbare Doppelkammerspritze (100), umfassend:
einen Zylinder (10), der eine zylindrische Form mit einer Achslinie (O) als ein Zentrum aufweist und einen Umgehungsabschnitt (11) aufweist, der durch eine Wölbung eines Teils einer inneren umfänglichen Oberfläche nach außen ausgebildet ist;
eine Luer-Lock Anlagefläche (20), die an dem vorderen Ende des Zylinders vorgesehen ist;
einen Fingergriff (60), der an dem hinteren Ende des Zylinders vorgesehen ist;
einen vorderen Stopper (30), der an der vorderen Stirnseite des Umgehungsabschnitts in den Zylinder eingepasst ist;
einen mittleren Stopper (40), der an der hinteren Stirnseite des Umgehungsabschnitts in dem Zylinder eingepasst ist, um ein Präparat (S) zusammen mit dem vorderen Stopper abzudichten;
einen Endstopper (50), der an der hinteren Stirnseite des mittleren Stoppers in dem Zylinder eingepasst ist, um ein Lösungsmittel zusammen mit dem mittleren Stopper abzudichten;
eine Kolbenstange (70), die mit dem Endstopper von der hinteren Stirnseite aus durch Einführen des Fingergriffs verbunden ist und die mit einem langen Stangenabschnitt vorgesehen ist, der sich entlang der Achslinie erstreckt, einem Verbindungsabschnitt, der an der vorderen Stirnseite des Stangenabschnitts vorgesehen ist, und eine an den Endstopper verbundene Schraubenform aufweist, und einem Druckabschnitt, der an der hinteren Stirnseite des Stangenabschnitts vorgesehen ist;
einen Innengewindeabschnitt (64), der ausgebildet ist, um die Achslinie an einer inneren umfänglichen Oberfläche des Fingergriffs herum geschraubt zuwerden; und
einen Schraubenabschnitt (73), der über einem vorbestimmten Bereich in der Richtung der Achslinie mehr auf der vorderen Stirnseite als ein mittlerer Abschnitt des Stangenabschnitts in der Richtung der Achslinie ausgebildet ist, und um mit dem Innengewindeabschnitt an einer äußeren umfänglichen Oberfläche der Kolbenstange verschraubbar zu sein,
**gekennzeichnet durch** eine Führungsnut (65), die an dem Innengewindeabschnitt ausgebildet ist und sich parallel zu der Achslinie erstreckt;
eine Führungsplatte (75), die an der hinteren Stirnseite des Schraubenabschnitts an der äußeren umfänglichen Oberfläche der Kolbenstange ausgebildet ist und in Einklang mit der Führungsnut geführt wird; und
einen ersten Vorsprung (66), der an dem hinteren Ende des Fingergriffs ausgebildet ist und mit dem die Führungsplatte in Kontakt gebracht werden kann, um in der Lage zu sein, diesen zu überwinden, wenn mindestens ein Teil des mittleren Stoppers, der durch Rotation der Kolbenstange in Einklang mit dem Innengewindeabschnitt und dem Schraubenabschnitt vorwärts geschoben werden kann, sich über den Umgehungsabschnittbewegt.

2. Vorfüllbare Doppelkammerspritze nach Anspruch 1, wobei das vordere Ende des Schraubenabschnitts dazu ausgelegt ist, das hintere Ende des Innengewindeabschnitts zu erreichen und in diesen geschraubt werden kann, wenn der mittlere Stopper, der durch Drücken der Kolbenstange vorgeschoben wird, in den Umgehungsabschnitteintritt.

3. Vorfüllbare Doppelkammerspritze nach Anspruch 1 oder 2, wobei die Verschraubung zwischen dem Schraubenabschnitt und dem Innengewindeabschnitt dazu ausgelegt ist, nachdem die Führungsplatte den ersten Vorsprung überwindet, durch Rotieren der Kolbenstange in Einklang mit dem Innengewindeabschnitt und dem Schraubenabschnittfreigegeben zu werden.

4. Vorfüllbare Doppelkammerspritze nach Anspruch 3, weiter umfassend:
einen zweiten Vorsprung (67), der an dem hinteren Ende des Fingergriffs ausgebildet ist und dazu ausgelegt ist, in einer Position in Kontakt gebracht zu werden, in der die Führungsplatte, die den ersten Vorsprung überwindet, in die Führungsnut eingepasst werdenkann.

5. Vorfüllbare Doppelkammerspritze nach einem der Ansprüche 1 bis 4, wobei das Präparat Aripiprazol beinhaltet.

## Revendications

1. Seringue à double compartiment préremplissable (100), comprenant :
un cylindre (10) qui a une forme cylindre avec une ligne d'axe (O) en tant que centre et a une partie de dérivation (11) formée en raison du renflement d'une partie d'une surface circonférentielle interne vers l'extérieur ;
un raccord luer-lock (20) qui est prévu au niveau de l'extrémité avant du cylindre ;
une prise pour les doigts (60) qui est prévue au niveau de l'extrémité arrière du cylindre ;
un bouchon avant (30) qui est ajusté sur le côté d'extrémité avant de la partie de dérivation dans le cylindre ;
un bouchon intermédiaire (40) qui est ajusté sur le côté d'extrémité arrière de la partie de dérivation dans le cylindre pour sceller une préparation (S) conjointement avec le bouchon avant ;
un bouchon d'extrémité (50) qui est ajusté sur le côté d'extrémité arrière du bouchon intermédiaire dans le cylindre pour sceller un solvant conjointement avec le bouchon intermédiaire ;
une tige de piston (70) qui est reliée au bouchon d'extrémité du côté d'extrémité arrière par l'insertion de la prise pour les doigts et qui est dotée d'une partie de tige longue s'étendant le long de la ligne d'axe, une partie de raccordement qui est ménagée sur le côté d'extrémité avant de la partie de tige et a une forme de vis mâle reliée au bouchon d'extrémité ; et une partie de pression qui est ménagée sur le côté de l'extrémité arrière de la partie de tige ;
une partie de vis femelle (64) qui est formée pour être vissée autour de la ligne d'axe sur une surface circonférentielle interne de la prise pour les doigts ; et
une partie de vis mâle (73) qui est formée sur une plage prédéterminée dans la direction de la ligne d'axe davantage sur le côté d'extrémité avant que sur une partie centrale de la partie de tige, dans la direction de la ligne d'axe, et pour pouvoir être vissée conjointement avec la partie de vis femelle sur une surface circonférentielle externe de la tige de piston,
**caractérisée par** une rainure de guidage (65) qui est formée dans la partie de vis femelle et s'étend parallèlement à la ligne d'axe ;
une plaque de guidage (75) qui est formée sur le côté d'extrémité arrière de la partie de vis mâle sur la surface circonférentielle externe de la tige de piston et est guidée conformément à la rainure de guidage ; et
une première saillie (66) qui est formée au niveau de l'extrémité arrière de la prise pour les doigts et avec laquelle la plaque de guidage est agencée pour être amenée en contact, de manière à pouvoir surmonter celle-ci, lorsqu'au moins une partie du bouchon intermédiaire, qui peut avancer en faisant tourner la tige de piston conformément à la partie de vis femelle et à la partie de vis mâle, traverse la partie de dérivation.

2. Seringue à double compartiment préremplissable selon la revendication 1, dans laquelle l'extrémité avant de la partie de vis mâle est agencée pour atteindre l'extrémité arrière de la partie de vis femelle, et peut être vissée sur celle-ci, lorsque le bouchon intermédiaire, qui est avancé en appuyant sur la tige de piston, entre dans la partie de dérivation.

3. Seringue à double compartiment préremplissable selon la revendication 1 ou la revendication 2, dans laquelle le vissage entre la partie de vis mâle et la partie de vis femelle est agencé de manière à être libéré après que la plaque de guidage surmonte la première saillie, en faisant tourner la tige de piston conformément à la partie de vis femelle et à la partie de vis mâle.

4. Seringue à double compartiment préremplissable selon la revendication 3, comprenant en outre :
une seconde saillie (67) qui est formée au niveau de l'extrémité arrière de la prise pour les doigts et est agencée pour être mise en contact à une position dans laquelle la plaque de guidage surmontant la première saillie peut être ajustée à la rainure de guidage.

5. Seringue à double compartiment préremplissable selon l'une quelconque des revendications 1 à 4, dans laquelle la préparation inclut de l'aripiprazole.
